# EUROPEAN PATENT SPECIFICATION

(11) **EP 1 834 611 B1**
(45) Date of publication and mention of the grant of the patent: **26.12.2012**
(21) Application number: 07004671.9
(22) Date of filing: 07.03.2007
(51) Int. Cl.: A61F 5/00

(54) **Transdermal magnetic coupling gastric banding**
Magenband mit transdermaler Magnetverbindung
Bandelette gastrique à couplage magnétique transdermique

(30) Priority: 13.03.2006 US 374573
(43) Date of publication of application: 19.09.2007
(73) Proprietor: Covidien LP, Mansfield, MA 02048 (US)
(72) Inventor: Scirica, Paul A., Huntington CT 06484 (US); Holsten, Henry E., Covington GA 30016 (US); Creston, Brian J., Milford CT 06460 (US); Viola, Frank J., Sandy Hook CT 06482 (US); Milliman, Keith L., Bethal CT 06801 (US)
(74) Representative: Soames, Candida Jane

(56) References cited:
- EP-A- 1 547 549
- WO-A-01/47575
- WO-A-2004/026399
- FR-A- 2 086 747
- US-A1- 2005 143 765
- US-B1- 6 331 744

## Description

### BACKGROUND

### 1. Technical field

The present disclosure relates to an adjustable surgical implant for treating obesity in a patient. More particularly, the present disclosure relates to a transdermally actuated adjustable surgical implant for constricting a portion of the digestive tract of a patient. FR-A-2086747 discloses the features of the pre-characterizing part of claim 1 below.

### 2. Background Of Related Art

Obesity is a leading cause of heart disease and many other serious illnesses i.e. diabetes, stroke. There are a variety of methods available for the treatment of obesity. Some of these rely on the willpower of the patient, which is usually used in the first instance. When this method fails however, surgical intervention is usually required. There are a variety of surgical procedures available to treat obesity. The more invasive of these typically include stapling of the stomach, gastric bypass surgery, and the insertion of a member, such as, for example, an inflatable or non-inflatable object into the stomach in order to reduce the capacity of the stomach.

Less invasive surgical procedures include positioning of a band about a portion of the stomach so as to reduce or restrict the flow of food through the stomach. These bands can be of the adjustable or nonadjustable type. The adjustable type of gastric band allows for adjustment of the restriction of the opening defined by the band so as to reduce or increase the flow of food through the stomach as the patient gains or loses weight. Several of these devices can be actuated by an external influence on an internal component of the device. One of these devices uses a powered internal component and an external component configured to actuate the powered internal component. However, the presence of power and associated electronics within the body could cause complications with other implants, such as, for example, pacemakers, and require regular battery charging or battery replacement. Thus, it would be desirable to have an adjustable gastric band system which can be actuated externally to adjust the gastric band without the use of any powered or electronic components.

For prior publications of transdermal magnetic coupling, see WO 2004/026399 and US-A-6331744. For other disclosures of gastric bands, see US-A-2005/0143765, WO 01/47575 and EP-A-1547549.

### SUMMARY

The invention is defined in claim 1 below.

The presently disclosed adjustable gastric banding assembly generally include an adjustable gastric band configured to be positioned about a portion of the digestive tract of the patient, a driver assembly operatively associated with the gastric band and a transmission assembly configured to effect movement between the driver assembly and the adjustable gastric band. The adjustable gastric band includes a portion which is generally fixed relative to the portion of the digestive tract about which it is mounted and a movable portion movable relative to the fixed portion so as to restrict or enlarge the opening defined by the gastric band.

The driver assembly includes an internal component or driver configured to be positioned within the patient and an external component or key operatively associated with the internal component. The external component is configured to transdermally move the internal component. The external and/or internal components include one or more magnetized members so as to effect movement of the internal component in response to movement of the external component. In one embodiment, both the internal and external components include one or more magnetized members of opposing polarities. The internal component is rotatably mounted within a housing. Manual rotation of the external component when positioned adjacent the internal component causes the internal component to rotate within the housing. The transmission assembly includes cable connected at its proximal end to the internal member and at its distal end to the movable portion of the gastric band. The transmission assembly includes a threaded screw at the distal end of the cable which is configured to engage corresponding structure on the adjustable portion of the gastric band. The corresponding structure on the adjustable portion of the gastric band includes a series of slots or teeth engageable with threads on the threaded screw such that rotation of the threaded screw causes translation of the movable portion of the gastric band relative to the fixed portion of the gastric band. The transmission assembly may further include an outer member surrounding the cable to protect surrounding tissue from movement of the cable. The threaded screw may be rotatably mounted within a housing affixed to a distal end of the outer member. A journal can be provided to stabilize the position of the threaded screw within the housing. The fixed portion of the gastric band can be affixed to the housing. Rotation of the internal component effects rotation of the cable within the outer member so as to rotate the threaded screw within its associated housing.

Typically magnets are mounted around a circumference of the internal and external components. The magnets are arranged asymmetrically about the circumference of the internal and external components. By providing varying arrangements of the magnets within the internal and external components, the drive assembly can be configured such that only one particular external component will affect the motion of the associated internal component. Such customization prevents unintentional or inadvertent adjustment of the gastric band without the associated external component. This provides a significant margin of safety for the patient.

In one method of use, the adjustable gastric band is positioned about the esophagus of the digestive tract. In an alternative method of use, the adjustable gastric band is positioned about a portion of the stomach.

### DESCRIPTION OF THE DRAWINGS

The invention of the presently disclosed gastric banding assembly is disclosed herein with reference to the drawings, wherein Figs. 1-4 and 6 do not represent the asymmetrical orientation of the fingers as defined in claim 1.
FIG. 1 is a perspective view of the presently disclosed transdermal magnetic coupling gastric banding device;
FIGS. 2A and B are side views, shown in section, of the banding device shown in FIG. 1;
FIG. 3 is a perspective view of the gastric banding device implanted in a patient;
FIGS. 4A and 4B are perspective views illustrating the gastric banding device being used to constrict the esophagus;
FIG. 5 is a perspective view of the driver and key having a different polarity configuration; and
FIG. 6 is a perspective view of the gastric banding device installed about a portion of the stomach of the patient.

### DETAILED DESCRIPTION OF EMBODIMENTS

The invention of the presently disclosed transdermal magnetic coupling gastric banding assembly will now be described in detail with reference to the drawings wherein like numerals designate identical or corresponding elements in each of the several views. As is common in the art, the term "proximal" refers to that part or component of the device closer to the user or operator, i.e. surgeon or physician, while the term "distal" refers to that part or component of the device further away from the user.

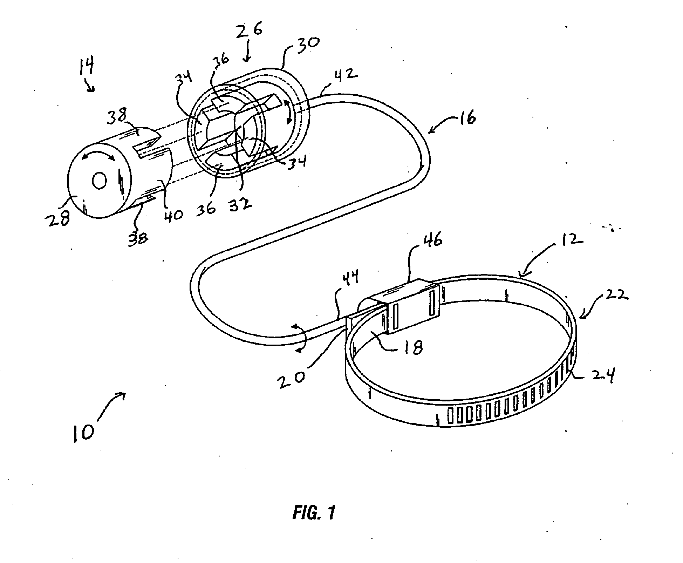

FIG. 1 illustrates the presently disclosed transdermal magnetic coupling gastric banding assembly (hereinafter "banding assembly") shown generally as 10. In general, banding assembly 10 includes an adjustable gastric band 12 and a drive assembly 14. A transmission assembly 16 is provided to interconnect adjustable gastric band 12 and drive assembly 14. Adjustable gastric band 12 generally includes a first end 18 secured to a portion of transmission assembly 16 and a second end 20 which is movable with respect to the portion of transmission assembly 16. Band 12 includes a drive portion 22 having drive slots 24. Drive slots 24 are provided to effect translation of second end 20 proximally and distally relative to transmission assembly 16.

Drive assembly 14 generally includes a driver 26 and a key 28 to move driver 26. Driver 26 includes a drive housing 30 and a magnet 32 movably mounted within drive housing 30. In particular, drive housing 30 is cylindrical and magnet 32 is rotatably mounted within drive housing 30. As shown, magnet 32 has fingers 34 which are of a first polarity and fingers 36 which are of a second polarity different from the first polarity. Similarly, key 28 includes magnetized fingers 38 which are of a first polarity and magnetized fingers 40 which are of a second polarity different from the first polarity. When properly positioned adjacent each other transdermally, i.e. through the skin, the fingers of magnet 32 and the fingers of key 28 which are of opposite polarity attract each other such that movement of key 28 in relation to magnet 32 effects movement of magnet 32 transdermally.

Transmission assembly 16 has a proximal end 42 which is connected to driver 26.

Transmission assembly 16 further includes a distal end 44 which is connected to first end 18 of band 12. A transmission housing 46 is provided at distal end 44 and is associated with second end 20 of band 12.

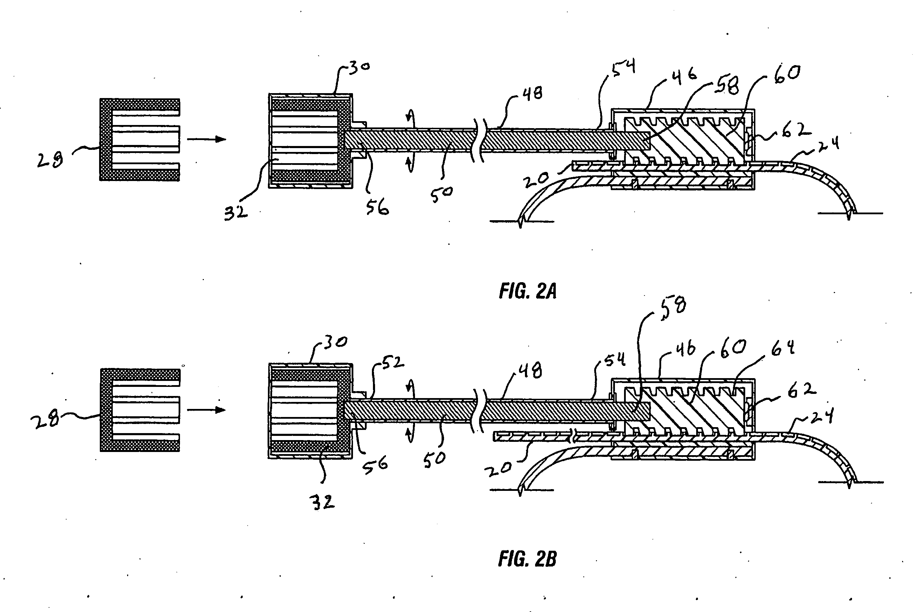

Referring also to FIGS. 2A and 2B, transmission assembly 16 includes an elongated outer member 48 and an inner member 50 rotatable within outer member 48. Specifically, a proximal end 52 of elongated outer member 48 is secured to drive housing 30. Similarly, a distal end 54 of elongated outer member 48 is secured to transmission housing 46. Inner member 50 includes a proximal end 56 and a distal end 58. Proximal end 56 of inner member 50 is secured to magnet 32.

A threaded screw 60 is provided within transmission housing 46 and is secured to distal end 58 of inner member 50. Threaded screw 60 is rotatably supported within housing 46 by a journal 62. Threads 64 provided on threaded screw 60 are configured to engage slots 24 in drive portion 22 of band 12. Thus, rotation of threaded screw 60 within housing 46 moves drive portion 22 proximally and distally relative to housing 46.

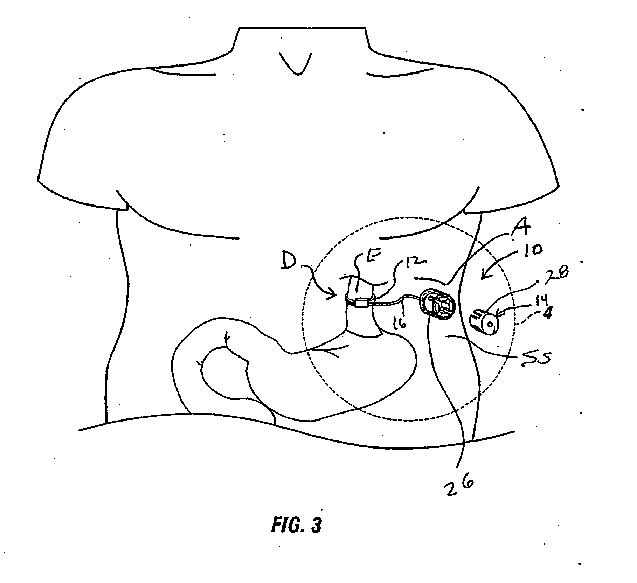

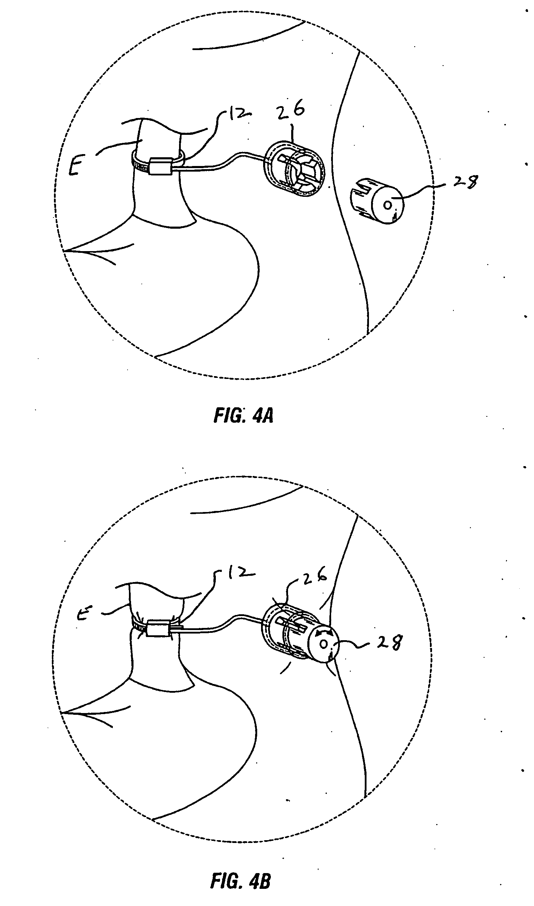

Referring now to FIGS. 3, 4A and 4B, and initially with reference to FIG. 3, the use of banding assembly 10 in a surgical gastric band procedure will now be described. Banding assembly 10 is provided to be positioned within the body of the patient to restrict a portion of the digestive tract. Initially, an incision is made through a portion of the abdominal wall "A" or chest cavity to access the digestive tract. As shown, band 12 is secured about the portion of the digestive tract "D" while driver 26 is positioned immediately under the skin surface "SS". As shown, key 28 remains outside the body. Key 28 is typically kept in the possession of the surgeon or treating physician to prevent deliberate, unintentional or inadvertent readjustment of band 12 by the patient. In FIG. 3 band 12 is illustrated as secured about a portion of the esophagus "E".

Referring now to FIGS. 4A and 4B, in order to adjust gastric band 12 about esophagus E, key 28 is positioned adjacent the location of driver 26. When key 28 is located sufficiently close to driver 26, the fingers of key 28 are attracted to the fingers of driver 26 having the polarity opposite those of the polarity of the fingers of key 26.

Referring for the moment to FIG. 2, rotation of key 28 relative to driver 26 effects rotation of magnet 32 within drive housing 30. Rotation of magnet 32 rotates inner member 50 within elongated outer member 48 thereby rotating threaded screw 60 within housing 46. As threaded screw 60 is rotated within housing 46, threads 64 engage slots 24 and move drive portion 22 within housing 46. Thus, in this manner, rotation of key 28 effects movement of second end 20 of band 12 so as to adjust band 12 about esophagus E to constrict or release the portion of esophagus E positioned within band 12. As noted above, this can be done repeatedly as the patient's weight changes without any further invasive surgery or use of any electronic components.

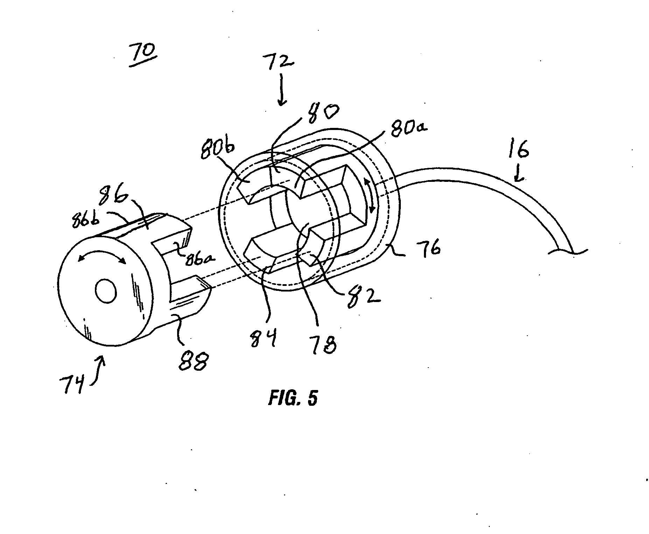

Referring now to FIG. 5, there is a disclosed an inventive embodiment of a drive assembly 70 for use with the above disclose gastric banding device 10. Drive assembly 70 includes a driver 72 and a key 74 to transdermally rotate driver 72. Driver 72 includes a drive housing 76 and a cylindrical magnet 78 which is rotatably mounted within drive housing 76. Cylindrical magnet 78 is connected to transmission assembly 16 in the manner described hereinabove with respect to drive assembly 14. Drive assembly 70 is provided to contain a plurality of magnets that are oriented asymmetrically. This will allow the surgeon to configure drive assembly 70 such that only a particular key 74 is capable of effecting rotation of cylindrical magnet 78 of driver 72.

Thus, for example, cylindrical magnet 78 may include a combination finger 80 and first and second fingers 82 and 84, respectively. Combination finger 80 can have first and second finger portions 80a and 80b, respectively, having the same or opposite polarities. Such as, for example, finger portion 80a can have a positive polarity while finger portion 80b can have a negative polarity. The polarities of first and second fingers 82 and 84, respectively, can also be the same or different.

Similarly, key 74 is provided with a combination finger 86 and first finger 88 and a second finger 90 (not shown). Like cylindrical magnet 78, combination finger 86 can have first and second finger portions 86a and 86b, respectively. In any given combination, the polarity of finger portion 80a will be opposite that of combination finger portion 86a such that finger portion 80a and finger portion 86a attracted other. The same would be true with finger portions 80b and 86b regardless of whether the polarities of each finger portion of the associated combination finger 86 are the same or opposite. Likewise, the polarity of first finger 82 is opposite the polarity of first finger 88 and the polarity of second finger 84 would be opposite that of the polarity of second finger 90. While only one combination finger and two individual fingers are illustrated and described herein with respect to each of the driver and key, is contemplated that multiple combination fingers and more or less than to individual fingers are specifically contemplated herein. Further, there need not be any combination fingers. However, in this regard, two or more individual fingers are arranged asymmetrically about the circumference of the associated driver and key.

As noted above, the locations of the combination fingers and the first and second fingers are asymmetrical about the circumference of the associated device to provide a relatively infinite amount of combinations of magnets so as to provide a true "key" function to the drive assembly.

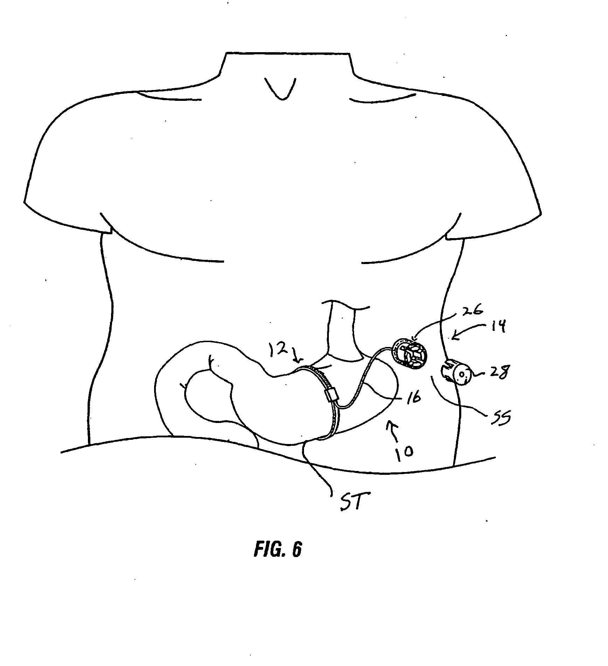

Banding device 10 can alternatively be used to constrict or reversibly restrict the flow of food through the stomach. As shown in FIG. 6, banding device 10 is implanted within the body of the patient such that band 12 is positioned around stomach "ST". Driver 26 is located just below the skin surface of the abdomen and connected to band 12 by transmission assembly 16.

As with the prior described use, key 28 remains outside the body and is used to effect movement of driver 26 such that band 12 constricts or expands around stomach "ST". Drive assembly 14 and drive assembly 70 are equally suitable for use with gastric banding device 10 in this procedure.

It will be understood that various modifications may be made to the embodiments disclosed herein. For example, numerous combinations of alternating magnets within the drive assembly may be provided to customize the banding device relative to a single patient to prevent interference thereof from any other source.

## Claims

1. A gastric banding assembly (10) to be operated through a transdermal magnetic coupling, and comprising:
an adjustable gastric band (12);
a drive assembly (14) having an internal component (26) and an external component (28), the external component being spaced from the internal component and being movable to effect corresponding movement of the internal component wherein the internal component is mounted within a housing (30) and rotation of the external component relative to the internal component rotates the internal component within the housing; and
a transmission assembly (16) that includes a cable for transferring movement of the internal component to the adjustable gastric band; and wherein
the internal component (26) adapted to be mounted or rotational movement within a body of a patient and operably connected to the cable to effect rotation of the cable;
a distal portion of the cable is operably connected to the gastric band such that rotation of the transmission assembly effects contraction or expansion of the gastric band, said operable connection to the gastric band comprising a threaded screw (60) on the distal portion of the cable, that is engageable with a moveable portion (22) of the gastric band, said threaded screw being engageable with slots (24) or teeth provided in the movable portion (22) of the gastric band such that rotation of the threaded screw in relation to the slots or teeth effects movement of the movable portion of the gastric band to contract or expand the gastric band about a portion (E) of the digestive tract (D) about which the gastric band is mountable; and the internal component and the external component include at least 3 magnetized fingers, the orientation of which, relative to each other, can be varied such that the internal component and the external component so configured will only function when both are present together, wherein the external component acts as a key to effect movement of the internal component within the body
**characterized in that**:
the gastric band is long enough to extend around the gastric tract from a first end (18) that is secured to the transmission assembly to a second end (20)which is movable with respect to the transmission assembly;
and the at least three magnetized fingers are oriented asymmetrically about a circumference of the internal or external component.

## Patentansprüche

1. Magenband-Baugruppe (10), zu bedienen über eine transdermale Magnetkupplung und umfassend:
ein verstellbares Magenband (12);
eine Antriebsbaugruppe (14) mit einem inneren Bauteil (26) und einem äußeren Bauteil (28), wobei das äußere Bauteil vom inneren Bauteil beabstandet ist und beweglich ist, um eine entsprechende Bewegung des inneren Bauteils zu bewirken, wobei das innere Bauteil innerhalb eines Gehäuses (30) angebracht ist und eine Drehung des äußeren Bauteils bezogen auf das innere Bauteil das innere Bauteil innerhalb des Gehäuses dreht; und
eine Übertragungsbaugruppe (16), die ein Kabel zum Übertragen von Bewegung des inneren Bauteils auf das verstellbare Magenband einschließt; und wobei
das innere Bauteil (26) so angepasst ist, dass es für Drehbewegung innerhalb eines Körpers eines Patienten angebracht und bedienbar mit dem Kabel verbunden ist, um eine Drehung des Kabels zu bewirken;
ein distaler Abschnitt des Kabels bedienbar mit dem Magenband verbunden ist, derart dass eine Drehung der Übertragungsbaugruppe eine Zusammenschnürung oder Lockerung des Magenbands bewirkt, wobei die bedienbare Verbindung mit dem Magenband eine Gewindeschraube (60) am distalen Abschnitt des Kabels umfasst, die mit einem beweglichen Abschnitt (22) des Magenbands in Eingriff bringbar ist, wobei die Gewindeschraube mit im beweglichen Abschnitt (22) des Magenbands vorgesehenen Schlitzen (24) oder Zähnen in Eingriff bringbar ist, derart dass eine Drehung der Gewindeschraube bezogen auf die Schlitze oder Zähne eine Bewegung des beweglichen Abschnitts des Magenbands bewirkt, um das Magenband um einen Abschnitt (E) des Verdauungstrakts (D), um den herum das Magenband anbringbar ist, zusammenzuschnüren oder zu lockern; und das innere Bauteil und das äußere Bauteil mindestens drei magnetisierte Finger einschließen, deren Ausrichtung bezogen aufeinander derart variiert werden kann, dass das so ausgestaltete innere Bauteil und äußere Bauteil nur funktionieren, wenn beide zusammen vorhanden sind, wobei das äußere Bauteil als Schlüssel zum Bewirken von Bewegung des inneren Bauteils innerhalb des Körpers wirkt,
**dadurch gekennzeichnet, dass**
das Magenband lang genug ist, um sich um den Magentrakt zu erstrecken, von einem ersten Ende (18), das an der Übertragungsbaugruppe befestigt ist, zu einem zweiten Ende (20), das bezogen auf die Übertragungsbaugruppe beweglich ist; und die mindestens drei magnetisierten Finger asymmetrisch um einen Umfang des inneren oder äußeren Bauteils ausgerichtet sind.

## Revendications

1. Ensemble de bandelette gastrique (10) susceptible d'opérer via un couplage magnétique transdermique et comprenant :
une bandelette gastrique réglable (12) ;
un ensemble d'entraînement (14) ayant un composant interne (26) et un composant externe (28), le composant externe étant espacé du composant interne et pouvant se déplacer pour assurer un mouvement correspondant du composant interne, dans lequel le composant interne est monté dans un boîtier (30) et la rotation du composant externe par rapport au composant interne fait tourner le composant interne dans le boîtier ; et
un ensemble de transmission (16) qui comprend un câble pour transférer le mouvement du composant interne à la bandelette gastrique réglable ; et dans lequel :
le composant interne (26) est à même d'être monté pour effectuer un mouvement de rotation dans le corps d'un patient et raccordé en service au câble pour effectuer la rotation du câble ;
une portion distale du câble est raccordée en service
à la bandelette gastrique de sorte que la rotation de l'ensemble de transmission entraîne la contraction ou l'expansion de la bandelette gastrique, ledit raccordement opératoire de la bandelette gastrique comprenant une vis filetée (60) sur la portion distale du câble, qui est à même de s'engager sur une portion mobile (22) de la bandelette gastrique, ladite vis filetée étant à même de s'engager dans des fentes (24) ou sur des dents prévues dans la portion mobile (22) de la bandelette gastrique de sorte que la rotation de la vis filetée par rapport aux fentes ou aux dents assure le mouvement de la portion mobile de la bandelette gastrique pour contracter ou dilater la bandelette gastrique autour d'une portion (E) de la voie digestive (D) autour de laquelle la bandelette gastrique peut être montée ; et le composant interne et le composant externe comprennent au moins trois doigts magnétisés dont l'orientation, l'un par rapport à l'autre, peut être modifiée afin que le composant interne et le composant externe ainsi configurés ne fonctionnent que lorsque tous deux sont présents conjointement, dans lequel le composant externe joue le rôle d'une clé pour assurer le mouvement du composant interne dans le corps,
**caractérisé en ce que** :
la bandelette gastrique est assez longue pour s'étendre autour de la voie gastrique d'une première extrémité (18), qui est fixée à l'ensemble de transmission, à une seconde extrémité (20) qui est mobile par rapport à l'ensemble de transmission ; et les au moins trois doigts magnétisés sont orientés de manière asymétrique autour d'une circonférence du composant interne ou externe.
